(19) Europäisches Patentamt
European Patent Office
Office européen des brevets

(11)  **EP 3 653 234 B1**

(12)  **EUROPEAN PATENT SPECIFICATION**

(45) Date of publication and mention
of the grant of the patent:
**04.09.2024  Bulletin 2024/36**

(21) Application number: **18831904.0**

(22) Date of filing: **05.07.2018**

(51) International Patent Classification (IPC):
*A61L 31/06* *(2006.01)*      *A61L 31/14* *(2006.01)*
*A61F 2/844* *(2013.01)*      *A61F 2/915* *(2013.01)*

(52) Cooperative Patent Classification (CPC):
(C-Sets available)
**A61F 2/915; A61L 31/06; A61L 31/14;**
**A61L 31/148;** A61F 2002/91583; A61L 2400/16

(Cont.)

(86) International application number:
**PCT/JP2018/025582**

(87) International publication number:
**WO 2019/013101 (17.01.2019 Gazette 2019/03)**

(54) **SELF-EXPANDABLE STENT AND METHOD FOR MANUFACTURING SAME**

SELBSTEXPANDIERBARER STENT UND VERFAHREN ZUR HERSTELLUNG DAVON

ENDOPROTHÈSE AUTO-EXPANSIBLE ET SON PROCÉDÉ DE FABRICATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **14.07.2017  JP 2017138397**

(43) Date of publication of application:
**20.05.2020  Bulletin 2020/21**

(73) Proprietor: **Terumo Kabushiki Kaisha**
**Tokyo 151-0072 (JP)**

(72) Inventors:
• **MATSUSHITA, Shuhei**
**Kanagawa 259-0151 (JP)**
• **TANI, Kazuyoshi**
**Kanagawa 259-0151 (JP)**

(74) Representative: **Casalonga**
**Casalonga & Partners**
**Bayerstraße 71/73**
**80335 München (DE)**

(56) References cited:
**WO-A1-2016/168706      JP-A- 2013 526 649**
**JP-A- 2016 512 063      US-A1- 2010 152 831**
**US-A1- 2015 252 144**

EP 3 653 234 B1

(52) Cooperative Patent Classification (CPC): (Cont.)

C-Sets
**A61L 31/06, C08L 67/04**

**Description**

Technical Field

The present invention relates to a self-expandable stent and a method of producing the same.

Background Art

Stent is a medical device that is used for expanding a stenosed or obstructed site to secure a lumen in order to treat various diseases caused by stenosis or obstruction of a lumen, such as a blood vessel. In recent years, acute myocardial infarction (AMI) has been also treated using a stent. In a treatment of AMI which is a thrombotic lesion using a stent, incomplete stent apposition (ISA) of the stent to a vascular wall is likely to occur due to thrombolysis after indwelling the stent.

Note that stents include balloon-expandable stents that are expanded with a balloon to which the stent is mounted and self-expandable stents that expand by itself by removal of a member that suppresses the expansion from the outside.

A self-expandable stent is stored in a delivery system, such as a sheath, in a contracted state, and on reaching the site to be indwelled, the stent is released from restriction to thereby self-expand. Thus an expansion operation, which is performed when a balloon-expandable stent is indwelled, is not required. As such a self-expandable stent, a self-expandable stent formed of a super-elastic alloy, such as a nickel-titanium alloy, is commercially available in Europe . Through a treatment with such a self-expandable stent, short-term incomplete stent apposition in AMI treatments has been dramatically improved.

As a material for forming a stent, nickel-titanium or other super-elastic alloys, which have a strong radial force (expansion retention force in the radial direction), are effective in that a vascular wall is maintained in a given diameter during the treatment. However, since a strong radial force is applied to the vascular wall over a long time after the period of treatment, major adverse cardiac events (MACE), in particular, target lesion revascularization (TLR), in the medium to long-term clinical outcomes are sometimes inferior to the case of a balloon-expandable stent.

In view of the above situation, a stent formed of a biodegradable material has been developed. Since a biodegradable material is gradually degraded in a living body, it is supposed that the radial force of the stent decreases over time to improve the medium to long-term clinical outcomes (particularly TLR) .

As such a biodegradable stent, JP-T-2015-527920 (WO 2014/018123) discloses a stent formed of a shape-memory random copolymer composed of poly (L-lactide) (PLLA) and a rubber-like polymer. Implantable medical devices comprising a biodegradable multiblock copolymer are also described in US 2010/152831.

In addition, US Patent Application Publication No. 2010/0262223 discloses a method of producing a stent, the method including crosslinking a biodegradable polymer by a crosslinking agent to form a base material.

Summary of Invention

However, the stent disclosed in JP-T-2015-527920 (WO 2014/018123) requires expansion with a balloon catheter in order to change the crimped state (contracted state) to the expanded state. Furthermore, [0119] in JP-T-2015-527920 (p38,12-8 in WO 2014/018123) has a statement that a stent fabricated using a resin composed of 90 : 10 (by mole) of polylactic acid and polycaprolactone inwardly recoils for 60 minutes after expansion and then outwardly recoils (recovers the shape) over several days. When the shape recovery is so slow as in the above case, incomplete stent apposition of the stent is likely to occur. The incomplete stent apposition of a stent may lead to onset of stent thrombosis and in some cases may lead to movement of the stent by blood flow. Accordingly, a rapid shape recovery for returning from a diameter in a contracted state to a diameter before contraction in a short period of time is required for a self-expandable stent.

In contrast, as disclosed in [0049] in JP-T-2015-527920 (p18, 114-21 in WO 2014/018123), an increased amount of a rubber-like polymer blended in a copolymer of PLLA and the rubber-like polymer is considered to lead to improvement of elastic properties of the polymer and reduction of the inward recoil. However, when the rubber-like polymer is merely increased in the resin, the stent does not have enough strength in the radial direction to support a stenosed artery.

In addition, US 2010/0262223 discloses a tendency of a stent to undergo self-expansion, but does not study about the speed of the shape recovery for outwardly expanding from a contracted state around body temperature (37°C). Furthermore, in US 2010/0262223, a self-crosslinking-type polymer formed of L-lactide and $\alpha$-allyl-$\sigma$-valerolactone or a self-crosslinking-type polymer formed of L-lactide-$\alpha,\alpha$-diallyl-$\sigma$-valerolactone is merely produced and no specific study is made about the specific characteristics thereof.

Furthermore, when a stent is contracted in diameter from an expanded state to a crimped state, a local stress of approximately 10% is applied to each of the tensile direction and the compression direction in the vicinity of the folding points (apexes of the zigzag) of a stent strut, generating a strain. Thus, resistance to the generated strain is also required.

Accordingly, an object of the present invention is to provide a self-expandable stent that has sufficient radial force, has sufficient strain resistance, and exhibits rapid shape recovery for outwardly expanding from a contracted state when released from restriction around body temperature (37°C) .

Solution to Problem

The present invention provides a self-expandable stent including a crosslinked polymer including a constitutional unit (A) derived from a monomer to constitute a rigid biodegradable polymer which is a homopolymer having a glass transition temperature (Tg) of 40 °C or more, a constitutional unit (B) derived from a monomer to constitute a rubber-like biodegradable polymer which is a homopolymer having a glass transition temperature (Tg) of 30 °C or lower, and a constitutional unit (C) derived from a crosslinking agent, the constitutional unit (C) being contained in an amount of 10% by weight or more and less than 60% by weight based on the total amount of the constitutional unit (A) and the constitutional unit (B) .

Brief Description of Drawings

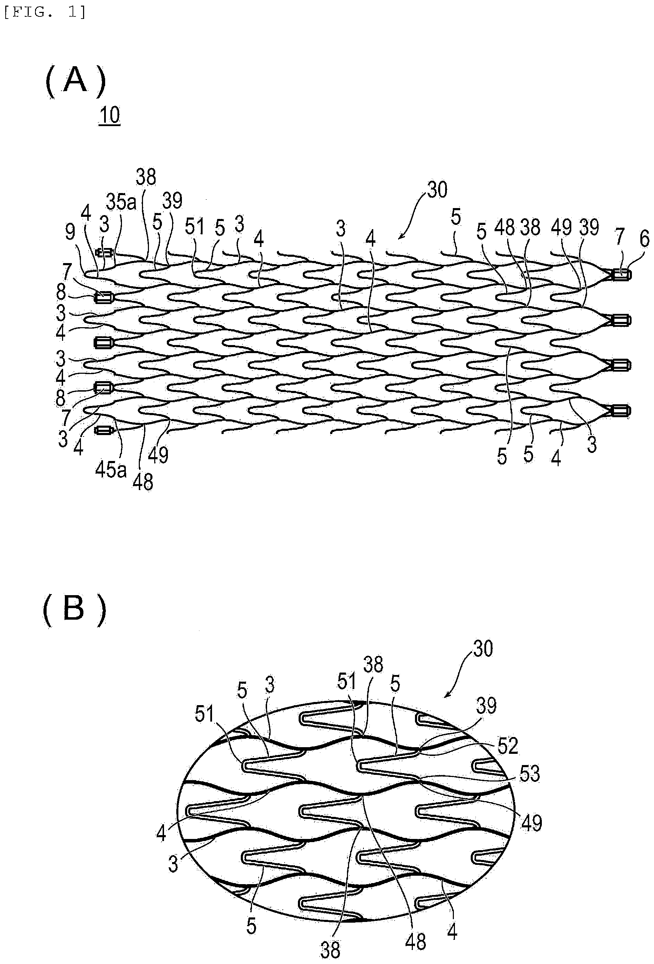

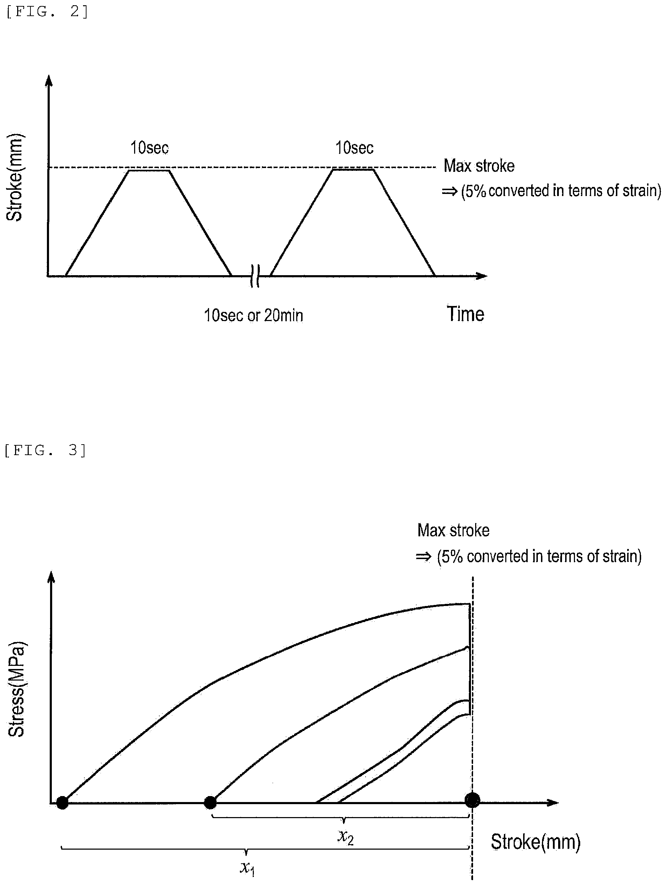

[Fig. 1] Fig. 1 is a diagram illustrating a stent according to an embodiment, wherein (A) is a development of the stent and (B) is a partial enlargement of (A). In Fig. 1, 3 denotes a first wave strut, 4 denotes a second wave strut, 5 denotes a connecting strut, 6 denotes a joint, 7 denotes a radiopaque marker, 8 denotes a bulging portion, 9 and 51 each denote a bent portion, 10 denotes a stent, 30 denotes a stent base, 35a and 45a each denote a slightly-bent portion, 38 denotes a top point of the first wave strut 3, 39 denotes a bottom point of the first wave strut 3, 48 denotes a bottom point of the second wave strut 4, and 49 denotes a top point of the second wave strut 4.
[Fig. 2] Fig. 2 is a graph showing the stroke displacement (stroke, length of displacement) over time in a tensile test for explaining a recovery rate.
[Fig. 3] Fig. 3 is a stress-stroke displacement chart in a tensile test for explaining a recovery rate.
[Fig. 4] Fig. 4 is a graph showing measurement results of radial force for stents of Example 25 and Comparative Example 7.

Description of Embodiments

Embodiments of the present invention will be described below. Note that the present invention is not limited only to the following embodiments. As used herein, "X to Y" for expressing a range means "X or more and Y or less" and unless otherwise specified, operations and measurements of physical properties are carried under conditions of room temperature (20 to 25°C) and a relative humidity of 40 to 50%RH.
A first embodiment of the present invention is a self-expandable stent that contains a crosslinked polymer containing a constitutional unit (A) derived from a monomer to constitute a rigid biodegradable polymer which is a homopolymer having a glass transition temperature (Tg) of 40 °C or more, a constitutional unit (B) derived from a monomer to constitute a rubber-like biodegradable polymer which is a homopolymer having a glass transition temperature (Tg) of 30 °C or lower, and a constitutional unit (C) derived from a crosslinking agent, the constitutional unit (C) being contained in an amount of 10% by weight or more and less than 60% by weight based on the total amount of the constitutional unit (A) and the constitutional unit (B).
The embodiment is capable of providing a self-expandable stent that rapidly recovers the shape after the stent is released from restriction in a contracted state, has sufficient resistance to local stress on a stent strut that is generated when the stent is contracted in diameter from an expanded state to a crimped state, and has increased radial force.
Hereinafter, the constitutional unit (A) derived from a monomer to constitute a rigid biodegradable polymer is referred to as a constitutional unit (A), the constitutional unit (B) derived from a monomer to constitute a rubber-like biodegradable polymer is as a constitutional unit (B), and the constitutional unit (C) derived from a crosslinking agent is as a constitutional unit (C).
The crosslinked polymer has a structure in which a copolymer containing the constitutional unit (A) and the constitutional unit (B) is crosslinked by a constitutional unit (C) derived from a crosslinking agent.
The constitutional unit (A), which is rigid, has rigidity around body temperature (37°C). The constitutional unit (B), which is like rubber, has elasticity around body temperature (37°C). Thus, the crosslinked polymer, which is a copolymer containing the constitutional unit (A) and the constitutional unit (B), has both properties of rigidity and elasticity (a property to return from a decreased diameter at insertion to a diameter before contraction) around body temperature.

However, it is difficult to achieve both the properties at high levels. In the embodiment, since a copolymer is crosslinked by a crosslinking agent, the high rigidity and elasticity can both be achieved. Furthermore, since the crosslinking agent is contained in an amount of 10% by weight or more and less than 60% by weight based on the total amount of the constitutional unit (A) and the constitutional unit (B), the stent has sufficient resistance to strain due to stress locally applied on a stent strut when the stent is contracted in diameter from an expanded state to a crimped state.

Thus, with such a structure, the stent can self-expand instantly (for example, within 10 seconds) from a diameter at insertion (a decreased diameter in a state where the stent is incorporated into a delivery system, for example, 1.5 mm) to an indwell diameter (a diameter immediately after indwell into a body, for example, 3.0 mm), and can further self-expand in a short time (for example, within 20 minutes) from the indwell diameter to the initial diameter (natural diameter in a state without restriction before incorporation into the delivery system, for example, 4.0 mm) . In addition, since a sufficient strength in the radial direction can be exhibited, the expansion in the radial direction of a wall of a lumen, such as a blood vessel, can be maintained while the strength in the radial direction of the stent decreases in the process of healing due to the biodegradability. It is therefore supposed that according to the self-expandable stent of the first embodiment, the incomplete stent apposition of a stent is reduced and, moreover, the radial force is reduced as a resin degrades over time, to improve the medium to long-term clinical outcomes (in particular, TLR).

The stent of the embodiment will be described below with reference to the drawings. Note that the ratios of sizes in the drawings are exaggerated for the sake of explanation and are sometimes different from the actual ratios. Note that in the Description, the longitudinal direction (right-left direction in Fig. 1(A)) of a stent is referred to as an axial direction.

First, components of the stent will be described. As an example of stent, a stent of Figs. 7 and 8 of WO 2011/034009 (US 2012/158119) can be mentioned. Note that the structure of a stent 10 explained by illustration in the drawings is only an example and the stent of the present invention is not limited to the shape and structure (for example, arrangement and design of a strut) explained here.

As shown in Figs. 1(A) and (B), the stent 10 according to the embodiment includes a stent base (stent body) 30 and is formed in a contour of a substantially cylindrical shape having a prescribed length in the axial direction as a whole. The stent 10 is indwelled in a lumen (for example, blood vessel, bile duct, trachea, esophagus, other gastrointestinal tracts, and urethra) in a living body and is used for expanding the lumen to treat a stenosed or obstructed site. The stent 10 is a self-expandable stent that self-expands so that a stent base 30 becomes to have a previously-memorized shape with a prescribed expanded diameter after the start of the indwell. In addition, the stent 10 is a biodegradable stent which is degraded and absorbed in a living body. A strut which forms the stent base 30 of the stent 10 is formed of a biodegradable crosslinked polymer. The crosslinked polymer is degraded by, for example, hydrolysis, in a living body.

The stent base 30 includes multiple wave struts 3 and 4 that extend in the axial direction from one end to the other end of the stent base 30 and that are arranged in the peripheral direction of the stent and multiple connecting struts 5 that connect adjacent wave struts 3 and 4. The adjacent wave struts 3 and 4 have multiple closer portions and distant portions and the connecting strut 5 connects the closer portions of the adjacent wave struts 3 and 4 and has, at a central portion, a bent portion 51 extending in the axial direction of the stent. The bent portion 51 of the connecting strut 5 is a free end extending toward the distal end of the stent 10. The first wave struts 3 and the second wave struts 4 have a shape of sine wave.

In the stent base 30, the first wave struts 3 and the second wave struts 4 have substantially the same wavelengths and substantially the same amplitudes, and the second wave struts 4 are shifted by about one-half wavelength in the axial direction of the stent with respect to the first wave struts 3.

Thus, as shown in Fig. 1(B), in the adjacent first wave strut 3 and second wave strut 4, the top point 38 or the bottom point 39 of the first wave strut 3 and the bottom point 48 or the top point 49 of the second wave strut 4 substantially face each other to form the closer portion and the distant portion. In the stent base 30, the wave struts 3 and 4 all have the same length except for the two ends.

In the stent base 30, two ends 52 and 53 of the connecting strut 5 that are connected to the wave struts 3 and 4 are slightly-bent portions which curve outside the connecting strut 5. The connecting strut 5 is connected to the top point 38 or the bottom point 39 of the wave strut 3 and to the bottom point 48 or the top point 49 of the wave strut 4 in the slightly-bent portions.

In the distal portion of the stent base 30, a bent portion 9 formed by connecting the distal portions of the first wave strut 3 and the second wave strut 4 and a bulging portion 8 provided in the bent portion 51 of the connecting strut 5 are alternately provided in the peripheral direction. A radiopaque marker 7 as described later is attached to the bulging portion 8. The bent portion 9 is positioned on the stent distal side with respect to the bulging portion 8. In this manner, a contrast marker on the distal side is positioned slightly on the inner side with respect to the stent end. Since the strut extends to an area outside the marker, the strut can securely cover a lesion area.

In the stent base 30, slightly-bent portions 35a and 45a which bend inside the bent portion 9 are provided at a given distance on the proximal side of the bent portion 9 formed by connecting the distal portions of the first wave strut 3 and the second wave strut 4 to thereby increase the expansion retention force of the bent portion 9 which is a long free end.

In the stent base 30, all the proximal portions of the first wave struts 3 and the second wave struts 4 are connected to joints 6 in the proximal portion of the stent. The stent base 30 has no free end that faces the proximal end of the stent except for the joints 6. In the other words, all the bent portions face the distal end of the stent. Thus, when a sheath is moved toward the distal end relative to the stent, the stent is not hooked on the sheath because of no free end facing the sheath (stent storage member) so that it is possible to re-store the stent in a sheath (stent storage member).

In addition, the radiopaque marker 7 is attached to the joint 6. The joint 6 includes two frame portions that extend toward the end in parallel with a given distance therebetween and the radiopaque marker 7 covers substantially all or a part of the two frame portions. The radiopaque marker 7 has a thin rectangular parallelepiped shape, stores the two frame portions therein, and is fixed to the two frame portions by means of a recessed center thereof . Examples of materials that can be suitably used for forming the radiopaque marker include one (single element) or two or more (alloy) selected from the group of elements consisting of iridium, platinum, gold, rhenium, tungsten, palladium, rhodium, tantalum, silver, ruthenium, and hafnium.

The stents according to the present invention include stents and stent grafts.

The thickness of the stent may be a conventionally common thickness. For example, the thickness of the stent is approximately from 50 to 500 $\mu$m, and in terms of the relationship between the supporting ability and the degradation time, the thickness is preferably approximately from 60 to 300 $\mu$m and more preferably approximately from 70 to 200 $\mu$m. Since the stent base according to the embodiment has superior physical properties (for example, expansion retention force), the thickness of the stent can be reduced.

The size of the stent is also appropriately adjusted according to the purpose and function thereof. For example, the outer diameter (the diameter) of the stent after expansion is preferably approximately from 1 to 40 mm, more preferably approximately from 1.5 to 10 mm, and particularly preferably approximately from 2 to 5 mm.

In addition, the length of the stent is not limited and can be appropriately selected depending on the disease to be treated. For example, the length is preferably approximately from 5 to 300 mm and more preferably approximately from 10 to 50 mm.

The stent base 30 is formed of a crosslinked polymer. The crosslinked polymer contains a constitutional unit (A) derived from a monomer to constitute a rigid biodegradable polymer which is a homopolymer having a glass transition temperature (Tg) of 40 °C or more, a constitutional unit (B) derived from a monomer to constitute a rubber-like biodegradable polymer which is a homopolymer having a glass transition temperature (Tg) of 30 °C or lower, and a constitutional unit (C) derived from a crosslinking agent. The crosslinked polymer has a structure in which polymer chains are crosslinked by the constitutional unit (C), and specifically, the crosslinked polymer is preferably produced by polymerizing a copolymer containing the constitutional unit (A) and the constitutional unit (B) with a crosslinking agent of 10% by weight or more and less than 60% by weight based on the copolymer.

Regarding the rigid biodegradable polymer, "a polymer that is rigid" means a homopolymer produced by polymerizing a monomer and having a glass transition temperature (Tg) of 40°C or higher. A polymer having a glass transition temperature of 40°C or higher has rigidity at body temperature (around 37°C). Thus, a copolymer having a monomer to constitute such a rigid biodegradable polymer can maintain a force in a radial direction even when indwelled in a lumen.

The glass transition temperature employed is a value measured by using Diamond DSC from Perkin Elmer according to JIS K7121:2012 (Testing Methods for Transition Temperatures of Plastics).

In addition, "biodegradable", as used herein, means that in a biodegradability test described in Examples, the elongation at fracture after a hydrolysis test is 90% or less (lower limit 0%) of the elongation at fracture before the hydrolysis test.

Specific examples of monomers to constitute a rigid biodegradable polymer include L-lactic acid (Tg of poly(L-lactic acid) (PLLA): 60°C), D-lactic acid (Tg of poly(D-lactic acid) (PDLA): 60°C), and glycolic acid (Tg of poly (glycolic acid) (PGA) : 45°C) . One of the monomers may be used alone or two or more thereof may be used in combination. For example, L-lactic acid and glycolic acid may be used in combination, and L-lactic acid and D-lactic acid may be used in combination.

Among them, because of superior biodegradability and mechanical strength, the monomer to constitute a rigid biodegradable polymer is preferably lactic acid and/or glycolic acid, more preferably lactic acid, and more preferably L-lactic acid.

Note that in the case of lactic acid, since a direct polycondensation method cannot give a high molecular weight polymer, a lactide, which can be polymerized by ring-opening polymerization optionally in the presence of a catalyst, may be used. Lactides include L-lactide which is a cyclic dimer of L-lactic acid, D-lactide which is a cyclic dimer of D-lactic acid, meso-lactide which is a cyclic dimer of D-lactic acid and L-lactic acid, and DL-lactide which is a racemic mixture of D-lactide and L-lactide. Also for glycolic acid, a direct polycondensation method cannot give a high molecular weight polymer, and thus ring-opening polymerization of glycolide can be used.

Regarding the rubber-like biodegradable polymer, "a polymer that is like rubber" refers to a homopolymer produced by polymerizing a monomer and having Tg of 30°C or lower. A polymer having a glass transition temperature of 30°C or lower has elasticity at body temperature (around 37°C). Thus, a stent comprise of a copolymer containing a monomer to constitute such a rubber-like biodegradable polymer can return from a diameter-decreased state in a delivery system to a diameter before contraction after indwell.

Examples of monomers to constitute a rubber-like biodegradable polymer include lactone monomers, such as $\epsilon$-caprolactone, $\sigma$-butyrolactone, and $\sigma$-valerolactone; hydroxyalkanoates, such as 4-hydroxybytyrate, 3-hydroxybytyrate, and 3-hydroxyvalerate; trimethylene carbonate, ethylene succinate, butylene succinate, and p-dioxanone. One of the monomers may be used alone or two or more thereof may be used in combination.

Among them, because of degradability into a low-toxic monomer, the monomer to constitute a rubber-like biodegradable polymer is preferably $\epsilon$-caprolactone (Tg of polycaprolactone -54°C), p-dioxanone (Tg of polydioxanone -10°C), or trimethylene carbonate (Tg of poly(trimethylene carbonate) -18°C), and more preferably $\epsilon$-caprolactone (Tg of polycaprolactone -54°C) or trimethylene carbonate (Tg of poly(trimethylene carbonate) -18°C), and more preferably $\epsilon$-caprolactone.

In a suitable aspect of the embodiment, the monomer to constitute a rigid biodegradable polymer is lactic acid and the monomer to constitute a rubber-like biodegradable polymer is $\epsilon$-caprolactone. Specifically, the copolymer containing the constitutional unit (A) and the constitutional unit (B) is preferably represented by $-(C_3H_4O_2)_n-(C_6H_{10}O_2)_m-$. Here, n and m represent molar fraction of the respective constitutional units in the polymer.

The constitutional ratio of the constitutional unit (A) and the constitutional unit (B) is not limited, but because of further rapid shape recovery and further increased radial force, the content of the constitutional unit (B) is preferably 10% by mole or more, more preferably 10 to 60% by mole, further preferably 10 to 35% by mole, furthermore preferably 10 to 30% by mole, and particularly preferably 20 to 30% by mole, based on the total amount of the constitutional unit (A) and the constitutional unit (B) . With a content of the constitutional unit (B) of 10% by mole or more, a stent that has improved elasticity and undergoes rapid shape recovery from a diameter at insertion can be obtained. With a content of the constitutional unit (B) of 60% by mole or less, the radial force can be secured. Note that the constitutional ratio of the constitutional units (A) and (B) is generally equal to the ratio of the respective monomers polymerized in production.

The copolymer containing the constitutional unit (A) and the constitutional unit (B) may be a random copolymer or may be a block copolymer. The block copolymer may be any of diblock (AB), triblock (ABA or BAB), multiblock (ABABA or BABAB), and the like.

The copolymer containing the constitutional unit (A) and the constitutional unit (B) may contain a constitutional unit derived from another monomer that is copolymerizable with the monomer to constitute a rubber-like biodegradable polymer and the monomer to constitute a rigid biodegradable polymer. In view of the effects of the present invention, such another monomer is preferably contained in an amount of 5% by weight or less, more preferably 2% by weight or less, and particularly preferably is substantially not contained.
"Substantially not contained" preferably means that the content is 0.01% by weight or less (lower limit 0% by weight).

Regarding a method of producing the copolymer containing the constitutional unit (A) and the constitutional unit (B), the copolymer can be produced by referring conventionally known methods. For example, when the constitutional unit (A) is lactic acid and the constitutional unit (B) is $\epsilon$-caprolactone, a polymerization reaction can be carried out using a lactide which is a cyclic dimer of lactic acid and $\epsilon$-caprolactone as starting materials in the presence of a metal catalyst. Examples of metal catalysts include tin chloride, tin octylate, zinc chloride, zinc acetate, lead oxide, lead carbonate, titanium chloride, titanium alkoxide, germanium oxide, and zirconium oxide. The polymerization reaction may be carried out in the presence of an organic solvent. An initiator may be used in the polymerization reaction. Lactides include L-lactide which is a cyclic dimer of L-lactic acid, D-lactide which is a cyclic dimer of D-lactic acid, meso-lactide which is a cyclic dimer of D-lactic acid and L-lactic acid, and DL-lactide which is a racemic mixture of D-lactide and L-lactide. Any lactide can be used in the present invention. In addition, two or more of the monomers may be combined to synthesize the copolymer.

From the viewpoints of improved mechanical strength and biodegradability, the copolymer preferably has a weight average molecular weight of 100,000 to 1,000,000, more preferably 150,000 to 800,000, and further preferably 150,000 to 600,000. Note that the weight average molecular weight in this description is a value measured by gel permeation chromatography (GPC) using polystyrenes as standard substances under the following measurement conditions.

(Conditions of molecular weight measurement)

    Apparatus: semi-micro GPC system LC-20AD (from Shimadzu Corporation)
    Detector: Shodex (registered tradename) RI-104 (from Showa Denko K.K.)
    Column: Shodex (registered tradename) GPC LF-404 (from Showa Denko K.K.)

Column temperature: 40°C
Mobile phase solvent: $CHCl_3$
Flow rate: 0.15 mL/min
Injection: 20 $\mu$L
Sample preparation: 2 mL of the mobile phase solvent is added to 6 mg of a sample to be measured to dissolve the sample, followed by filtering through a 0.45-$\mu$m PTFE membrane filter.

As the copolymer containing the constitutional unit (A) and the constitutional unit (B), a commercial product may be used, and examples of commercial products include Resomer (registered tradename) LC703S, Resomer (registered tradename) DLCL9010, and Resomer (registered tradename) LT706S (all from EVONIK Industries AG); and BioDegmer (registered tradename) LCL (75 : 25), BioDegmer (registered tradename) LCL (60 : 40), BioDegmer (registered tradename) LCL (50 : 50), BioDegmer (registered tradename) LCL (90 : 10), and BioDegmer (registered tradename) LCL (65 : 35) (all from BMG Inc.) .

A copolymer containing the constitutional unit (A) and the constitutional unit (B) is hereinafter also referred to as simply a copolymer.

The constitutional unit (C) is derived from a crosslinking agent.

The crosslinking agent in the constitutional unit (C) is preferably a monomer having two or more polymerizable unsaturated bond. Examples of polymerizable unsaturated bonds include an acryloyl group ($CH_2$=CH-CO-), a methacryloyl group ($CH_2$=C($CH_3$)-CO-), or a vinyl group ($CH_2$=CH-).

Specific examples of crosslinking agents include bifunctional (meth)acrylates, such as diethylene glycol diacrylate, 1,4-butanediol diacrylate, 1,3-butylene glycol diacrylate, dicyclopentanyl diacrylate, glycerol diacrylate, 1,6-hexanediol diacrylate, neopentyl glycol diacrylate, tetraethylene glycol diacrylate, ethylene glycol dimethacrylate, polyethylene glycol dimethacrylate, diethylene glycol dimethacrylate, triethylene glycol dimethacrylate, 1,4-butanediol dimethacrylate, 1,3-butylene glycol dimethacrylate, dicyclopentanyl dimethacrylate, glycerol dimethacrylate, 1,6-hexanediol dimethacrylate, neopentyl glycol dimethacrylate, tetraethylene glycol dimethacrylate, 1,9-nonanediol dimethacrylate, and 1,10-decanediol dimethacrylate; trifunctional (meth)acrylates, such as trimethylolpropane triacrylate, pentaerythritol triacrylate, tetramethylolmethane acrylate, trimethylolpropane trimethacrylate, and pentaerythritol trimethacrylate; tetra- or higher-functional (meth)acrylates, such as pentaerythritol tetraacrylate, ditrimethylolpropane tetraacrylate, dipentaerythritol hexaacrylate, dipentaerythritol monohydroxypentaacrylate, pentaerythritol tetramethacrylate, ditrimethylolpropane tetramethacrylate, dipentaerythritol hexamethacrylate, and dipentaerythritol monohydroxypentamethacrylate; N,N'-methylenebisacrylamide, N,N'-methylenebismethacrylamide, N,N'-ethylenebisacrylamide, N,N'-ethylenebismethacrylamide, N,N'-hexamethylenebisacrylamide, N,N'-hexamethylenebismethacrylamide, N,N'-benzylidenebisacrylamide, and N,N'-bis(acrylamidemethylene)urea; carboxylic acid allyl esters, such as trimellitic acid triallyl ester, pyromellitic acid triallyl ester, and diallyl oxalate; cyanuric acid or isocyanuric acid allyl esters, such as triallyl cyanurate and triallyl isocyanurate; maleimide compounds, such as N-phenylmaleimide and N,N'-m-phenylenebismaleimide; compounds having two or more triple bonds, such as dipropargyl phthalate and dipropargyl maleate; and divinylbenzene.

The crosslinking agent is more preferably a monomer having an acryloyl group ($CH_2$=CH-CO-) or a methacryloyl group ($CH_2$=C($CH_3$)-CO-) because of further improved shape recovery. That is, the crosslinking agent is preferably a multifunctional (meth)acrylate. Furthermore, because of superior initial radial force, the multifunctional (meth)acrylate as the crosslinking agent is further preferably a tetra- or higher-functional (meth)acrylate, and particularly preferably a tetra- to hexa-functional (meth)acrylate.

Among them, the crosslinking agent is preferably pentaerythritol tetraacrylate, ditrimethylol propane tetraacrylate, dipentaerythritol hexaacrylate, dipentaerythritol monohydroxypentaacrylate, pentaerythritol tetramethacrylate, ditrimethylolpropane tetramethacrylate, dipentaerythritol hexamethacrylate, and/or dipentaerythritol monohydroxypentamethacrylate, and more preferably pentaerythritol tetraacrylate and/or dipentaerythritol hexaacrylate.

In addition, the absolute value of the difference between the solubility parameter of the crosslinking agent and the weighted average of the solubility parameters of the constitutional unit (A) and the constitutional unit (B) is preferably 5 $(J/cm^3)^{1/2}$ or less. That is, in a suitable aspect of the first embodiment, the crosslinked polymer is produced by polymerizing the crosslinking agent with the copolymer containing the constitutional unit (A) derived from a monomer to constitute a rigid biodegradable polymer and the constitutional unit (B) derived from a monomer to constitute a rubber-like biodegradable polymer, and the absolute value of the difference between the solubility parameter of the crosslinking agent and the weighted average of the solubility parameters of the constitutional unit (A) and the constitutional unit (B) (hereinafter also referred to as solubility parameter difference) is 5 $(J/cm^3)^{1/2}$ or less. With a solubility parameter difference of 5 $(J/cm^3)^{1/2}$ or less, the effects of the present invention (rapid shape recovery (high recovery rate) and high radial force (high Young's modulus)) are more easily obtained. This is because high compatibility of the crosslinking agent and the copolymer leads to a uniform crosslinking reaction. The solubility parameter difference is more preferably 2 $(J/cm^3)^{1/2}$ or less and particularly preferably 1.5 $(J/cm^3)^{1/2}$ or less. Note that the lower limit of the solubility parameter difference is zero.

"Solubility parameter (SP value) " refers to an SP value determined by a formula based on the Fedors method. Specifically, the SP value can be calculated by the following formula (1) as described in Robert F Fedor, Poly Eng Sci 1974; 14(2): 147-154.

[Math. 1]

$$SP = (\Delta E_V/V)^{1/2}$$

In the formula, ΔEv represents the molar cohesive energy (the energy of vaporization at a given temperature) and V represents the molar volume. Note that in this application, "at a given temperature" means that the value was measured at 25°C.

The weighted average of the solubility parameters of the constitutional unit (A) and the constitutional unit (B) is determined as follows.

[Math. 2]

$$SP_{CO} = SP_A \times N_A + SP_B \times N_B$$

$SP_{CO}$: weighted average of solubility parameters of constitutional unit (A) and constitutional unit (B)
$SP_A$: SP value of constitutional unit (A)
$SP_B$: SP value of constitutional unit (B)
$N_A$: molar fraction of constitutional unit (A) in copolymer
$N_B$: molar fraction of constitutional unit (B) in copolymer

One of the crosslinking agents may be used alone or two or more thereof may be used in combination.

The content of the constitutional unit (C) is 10% by weight or more and less than 60% by weight based on the total amount of the constitutional unit (A) and the constitutional unit (B). With a content of the constitutional unit (C) less than 10% by weight based on the total amount of the constitutional unit (A) and the constitutional unit (B), the Young's modulus significantly decreases and the expansion force in the radial direction cannot be maintained (Comparative Example 4 or Comparative Example 5 described later). On the other hand, with a content of the constitutional unit (C) of 60% by weight or more based on the crosslinked polymer, the resin is brittle and the strain resistance is reduced (Comparative Example 3 described later). From the viewpoint of increased expansion force of a stent, the content of the constitutional unit (C) is preferably more than 10% by weight, more preferably 15% by weight or more, further preferably 20% by weight or more, and furthermore preferably 25% by weight or more, and particularly preferably 30% by weight or more based on the total amount of the constitutional unit (A) and the constitutional unit (B). From the viewpoint of the strain resistance, the content of the constitutional unit (C) is preferably 50% by weight or less, more preferably 45% by weight or less, and further preferably 40% by weight or less based on the total amount of the constitutional unit (A) and the constitutional unit (B). The content of the constitutional unit (C) is preferably 10 to 50% by weight, more preferably 10 to 45% by weight, further preferably 10 to 40% by weight, furthermore preferably 20 to 40% by weight, particularly preferably 25 to 40% by weight, and most preferably 30 to 40% by weight, based on the total amount of the constitutional unit (A) and the constitutional unit (B). When the crosslinking agent is a tetrafunctional (meth) acrylate, the content of the constitutional unit (C) is preferably 20 to 50% by weight and more preferably 30 to 50% by weight because of high Young's modulus, and particularly preferably 30 to 40% by weight in view of the balance between the shape recovery and radial force, based on the total amount of the constitutional unit (A) and the constitutional unit (B). When the crosslinking agent is a hexafunctional (meth)acrylate, the content of the constitutional unit (C) is preferably 30 to 50% by weight based on the total amount of the constitutional unit (A) and the constitutional unit (B) because of high Young's modulus.

Note that the content of the constitutional unit (C) corresponds to the amount of the crosslinking agent added in the production process. The content of the constitutional unit (C) can be known by hydrolytically degrading the stent material to the monomer constitutional units and quantifying the monomer containing the constitutional unit (C) by HPLC.

The crosslinked polymer can be produced by polymerizing the copolymer with the crosslinking agent of 10% by weight or more and less than 60% by weight based on the copolymer. The method of producing the crosslinked polymer will be described later.

Crosslink refers to a chemical bond to bind one polymer chain to another polymer chain. As a non-limiting example, a C-H bond in a copolymer is cut by, for example, ultraviolet irradiation to generate a free radical site, and the free radical site reacts with an unsaturated bonding site in a crosslinking agent, thereby forming a structure in which the copolymer is crosslinked with the crosslinking agent.

The crosslinked polymer may contain, in addition to the constitutional units (A), (B), and (C), another biodegradable constitutional unit. Examples of compounds used for introducing said another constitutional unit into a polymer include hydroxycarboxylic acids, dicarboxylic acids, polyhydric alcohols, and cyclic depsipeptide. The content of said another constitutional unit is preferably 0 to 10% by mole and more preferably 0 to 5% by mole based on all the constitutional units of the crosslinked polymer.

The Young's modulus of the crosslinked polymer is preferably 400 $N/mm^2$ or more, more preferably 500 $N/mm^2$ or more, and further preferably 550 $N/mm^2$ or more. With a Young's modulus within the above range, a high radial force is achieved and the mechanical strength is secured. The Young's modulus of the crosslinked polymer is further preferably 600 $N/mm^2$ or more, furthermore preferably 800 $N/mm^2$ or more, and particularly preferably 1000 $N/mm^2$ or more. A higher Young's modulus of the crosslinked polymer is more preferred. The upper limit is not limited but is generally 3000 $N/mm^2$ or less. The Young's modulus of the crosslinked polymer can be controlled by the amount of the crosslinking agent added, the type of the crosslinking agent (combination of the crosslinking agent and the copolymer), and the like. The larger the amount of the crosslinking agent added to the copolymer, the larger the Young's modulus.

As the Young's modulus of the crosslinked polymer, a value measured by a method described later in Examples is employed.

The crosslinked polymer preferably has a recovery rate after 10 seconds of 70% or more. With a recovery rate after 10 seconds of 70% or more, the diameter can immediately return from a decreased diameter at insertion to a diameter before contraction, and expansion by a balloon catheter is not required and incomplete stent apposition is reduced. The recovery rate after 10 seconds is more preferably 72% or more and further preferably 75% or more. The upper limit of the recovery rate after 10 seconds is 100%, but generally 95% or less. The recovery rate after 10 seconds can be controlled by the type and amount of the constitutional unit (B) and the type and amount of the constitutional unit (C). The higher the proportion of the constitutional unit (B), the higher the recovery rate after 10 seconds. Also, the higher the proportion of the constitutional unit (C), the higher the recovery rate after 10 seconds.

The recovery rate after 20 minutes of the crosslinked polymer is preferably 90% or more and more preferably 91.0% or more. With a recovery rate after 20 minutes of 90% or more, the stent can substantially return from a decreased diameter at insertion to a diameter before contraction and the incomplete stent apposition is reduced. The upper limit of the recovery rate after 20 minutes is 100%.

As the recovery rate after 10 seconds or the recovery rate after 20 minutes of the crosslinked polymer, a value measured by a method described later in Examples is employed.

In this description, a self-expansion stent refers to a stent in which the recovery rate after 20 minutes of the resin forming the stent base (for example, crosslinked polymer) is 70% or more.

In a suitable aspect of the embodiment, the Young's modulus of the crosslinked polymer is 500 $N/mm^2$ or more and the recovery rate after 10 seconds is 70% or more. In another suitable aspect of the embodiment, the Young's modulus of the crosslinked polymer is 600 $N/mm^2$ or more and the recovery rate after 10 seconds is 70% or more. In still another suitable aspect of the embodiment, the Young's modulus of the crosslinked polymer is 800 $N/mm^2$ or more and the recovery rate after 10 seconds is 70% or more. In still another suitable aspect of the embodiment, the Young's modulus of the crosslinked polymer is 800 $N/mm^2$ or more and the recovery rate after 10 seconds is 72% or more.

The glass transition temperature (Tg) of the crosslinked polymer is preferably 45°C or lower and more preferably 40°C or lower. With a glass transition temperature of the crosslinked polymer within the above temperature range, such a stent has elasticity at a temperature around body temperature and therefore exhibits high recovery speed. Note that a lower glass transition temperature is more preferred, but the glass transition temperature is generally -50°C or higher.

The crosslinked polymer has a gel fraction of 50% or more and more preferably 60% or more. With a gel fraction within the above range, the crosslinking sufficiently proceeds and a desired effect can be achieved. That is, the degree of crosslinking can be known through the gel fraction. The upper limit of the gel fraction is not limited, but is preferably 100% or less. As the gel fraction, a value measured by a method described later in Examples is employed.

Alternatively, the degree of crosslinking (for example, crosslinking density) in the crosslinked polymer can be measured by a method of tracing the degree of decrease in the peak of the heat of fusion by DSC.

Furthermore, the stent is preferably has a Martens hardness in a loading-unloading test using a nanoindenter (hereinafter also referred to as simply Martens hardness) of 50 $N/mm^2$ or more. With a Martens hardness of 50 $N/mm^2$ or more, a high radial force is achieved and the mechanical strength is secured. The Martens hardness is preferably 100 $N/mm^2$ or more. A stent having a higher Martens hardness is more preferred, and the upper limit is generally, but not limited to, 200 $N/mm^2$ or less. The Martens hardness of the stent can be controlled by the amount of the crosslinking agent added, the type of the crosslinking agent (combination of the crosslinking agent and the copolymer), and the like. The larger the amount of the crosslinking agent added to the base polymer (copolymer), the larger the Martens hardness.

The crosslinked polymer preferably has lower crystallinity (higher amorphousness) in view of easy hydrolysis, namely, biodegradability. Thus, any operation for increasing the crystallinity, such as annealing, is not required in the production process.

A second embodiment of the present invention is a method of producing a self-expandable stent including polymerizing a copolymer that contains a constitutional unit (A) derived from a monomer to constitute a rigid biodegradable polymer and a constitutional unit (B) derived from a monomer to constitute a rubber-like biodegradable polymer and a crosslinking agent of 10% by weight or more and less than 60% by weight based on the copolymer to produce a crosslinked polymer, and fabricating the stent using the crosslinked polymer.

The constitutional unit (A) derived from a monomer to constitute a rigid biodegradable polymer and the constitutional unit (B) derived from a monomer to constitute a rubber-like biodegradable polymer, and the copolymer containing the units are as described above.

In addition, specific examples of the crosslinking agents are as described above.

The polymerization of the copolymer and the crosslinking agent may be any mode, such as solution polymerization or bulk polymerization, but not limited thereto. The solvent used in the solution polymerization may be a solvent that can dissolve the copolymer and the crosslinking agent, and examples thereof include chloroform, 1,1,1,3,3,3-hexafluoro-2-propanol, and N-N-dimethylformamide.

The method of polymerization is preferably photopolymerization since unsaturated bonds can be easily activated. Examples of light (active radiations) used herein include ionizing radiations, such as electron beams, α-rays, β-rays, and γ-rays; and ultraviolet rays. Among them, the polymerization of a polymer and a crosslinking agent is preferably carried out under irradiation with ultraviolet rays because of simple production facility and easy production. The wavelength of the ultraviolet rays is preferably 200 to 400 nm. The quantity of the ultraviolet rays (integral light quantity) is appropriately set so that the polymerization is suitably achieved, and, for example, the quantity is 500 to 20,000 $mJ/cm^2$ and preferably is 1,000 to 5,000 $mJ/cm^2$.

For enhancing polymerization efficiency, such polymerization under irradiation with ultraviolet rays is preferably carried out in the presence of photoinitiator. The photoinitiator may be selected depending on the wavelength of the ultraviolet rays used, and may be benzyldimethylketal, any of alkylphenone compounds, such as α-hydroxyalkylphenones and α-aminoalkylphenones; acylphosphine oxide compounds, such as MAPO and BAPO; and oxime ester compounds . From the viewpoint of polymerization efficiency, an alkylphenone compound is preferred and an α-hydroxyalkylphenone is more preferred.

Specific examples of photoinitiators include α-hydroxyalkylphenones, such as 2-hydroxy-1-[4-(2-hydroxyethoxy)phenyl]-2-methyl-1-propano ne, 2-hydroxy-2-methyl-1-phenyl-1-propanone, and 1-hydroxy-cyclohexyl-phenyl-ketone; α-aminoalkylphenones, such as 2-methyl-1-[4-methylthiophenyl]-2-morpholinopropan-1-one, 2-benzyl-2-dimethylamino-1-(4-morpholinophenyl)butanone-1, and 2-dimethylamino-2-(4-methylbenzyl)-1-(4-morpholin-4-ylphen yl)butan-1-one; and acylphosphineoxide compounds, such as diphenyl(2,4,6-trimethylbenzoyl)-phosphineoxide and phenylbis(2,4,6-trimethylbenzoyl) .

As the photoinitiator, a commercial product may be used, and examples of commercial products include Irgacure (registered tradename) 2959, 184, 1173, 907, 369E, 379EG, TPO, and 819 (all from BASF).

One of the photoinitiators may be used alone or two or more thereof may be used in combination.

The amount of the photoinitiator is preferably 1 to 20% by weight and more preferably 2 to 15% by weight based on the amount of the crosslinking agent.

The timing of irradiation with light is not limited and the irradiation may be carried out after molding a mixture containing the copolymer and the crosslinking agent into a tube shape by extrusion or injection molding. Note that processing into a desired stent shape may be achieved by laser cutting or the like. Furthermore, irradiation with light may be performed after molding a mixture containing the copolymer and the crosslinking agent into a tube shape by extrusion or injection molding and then processing the obtained tube into a desired stent shape by laser cutting or the like. Alternatively, irradiation with light may be performed after processing a mixture containing the copolymer and the crosslinking agent into a stent shape by injection molding or the like.

In the stent, any component other than the crosslinked polymer may be contained to the extent that the object and effects of the present invention are not impaired. An example of said other component is a drug for suppressing stenosis or obstruction of the vascular system which possibly occurs when a stent is indwelled in a lesion area. Specific examples include anticancer agents, immunosuppressive agents, antibiotics, antithrombotic agents, HMG-CoA reductase inhibitors, ACE inhibitors, calcium antagonists, antilipemic agents, integrin inhibitors, antiallergic agents, antioxidants, GPIIbIIIa antagonists, retinoids, lipid improvers, antiplatelet agents, and anti-inflammatory agents. These drugs have an advantage of capability of controlling behavior of tissue cells in a lesion area to thereby treat the lesion area. Said other component as described above may constitute a stent base together with the crosslinked polymer or may be present as a coating layer over a stent base formed by the crosslinked polymer.

Preferred examples of anticancer agent include, but not limited to, paclitaxel, docetaxel, vinblastine, vindesine, irinotecan, and pirarubicin.

Preferred examples of immunosuppressive agents include, but not limited to, sirolimus derivatives, such as sirolimus, everolimus, pimecrolimus, and zotarolimus, biolimus (for example, Biolimus A9 (registered tradename)), tacrolimus, azathioprine, cyclosporine, cyclophosphamide, mycophenolate mofetil, and gusperimus.

Preferred examples of antibiotics include, but not limited to, mitomycin, adriamycin, doxoruvicin, actinomycin, daunorubicin, idarubicin, pirarubicin, aclarubicin, epirubicin, and zinostatin stimalamer.

Preferred examples of antithrombotic agents include, but not limited to, aspirin, ticlopidine, and argatroban.

Preferred examples of HMG-CoA reductase inhibitors include, but not limited to, cerivastatin, cerivastatin sodium, atorvastatin, pitavastatin, fluvastatin, fluvastatin sodium, simvastatin, and lovastatin.

Preferred examples of ACE inhibitors include, but not limited to, quinapril, trandolapril, temocapril, delapril, enalapril maleate, and captopril.

Preferred examples of calcium antagonists include, but not limited to, nifedipine, nilvadipine, benidipine, and nisoldipine.

A preferred example of an antilipemic agent is, but not limited to, probucol.

A preferred example of an integrin inhibitor is, but not limited to, AJM300.

A preferred example of an antiallergic agent is, but not limited to, tranilast.

Preferred examples of antioxidants include, but not limited to, $\alpha$-tocopherol, catechin, dibutylhydroxytoluene, and butylhydroxyanisole.

A preferred example of a GPIIbIIIa antagonist is, but not limited to, abciximab.

A preferred example of a retinoid is, but not limited to, all trans retinoic acid.

A preferred example of a lipid improver is, but not limited to, eicosapentaenoic acid.

Preferred examples of antiplatelet agents include, but not limited to, ticlopidine, cilostazol, and clopidogrel.

Preferred examples of anti-inflammatory agents include, but not limited to, steroids, such as dexamethasone and prednisolone.

When the stent contains any components other than the crosslinked polymer, the crosslinked polymer is contained in an amount of 80% by weight or more, preferably 90% by weight or more, more preferably 95% by weight or more (upper limit 100% by weight) in total based on the whole stent, and the balance is the other components.

The stent according to the present invention may be provided with a coating layer of any biodegradable material on a stent base in addition to the stent base to the extent that the object and effects of the present invention are not impaired. Examples biodegradable materials used for forming the coating layer include, but not limited to, a polymer selected from the group consisting of polyesters, polyacid anhydrides, polycarbonates, polyphosphazenes, polyphosphoric acid esters, polypeptides, polysaccharides, proteins, and celluloses. Specific examples include at least one or a blend of two or more selected from the group consisting of polylactic acids, polyglycolic acids, lactic acid-glycolic acid copolymers, polycaprolactone, lactic acid-caprolactone copolymers, polyhydroxybutyric acid, polymalic acid, poly-$\alpha$-amino acids, collagen, laminin, heparan sulfate, fibronectin, vitronectin, chondroitin sulfate, and hyaluronic acid, and medically safe ones are preferred in view of degradability in living bodies. The duration of strength can be prolonged by adjusting the molecular weight, degree of purification, degree of crystallization of the biodegradable material which coats a stent outer surface (stent base outer surface) to suppress the hydrophilicity. For example, the time for hydrolysis can be prolonged, for example, by increasing the degree of purification of the biodegradable material to eliminate unreacted monomers and low molecular weight fractions or by increasing the degree of crystallization to suppress the amount of water permeating the inside of the stent backbone. The coating layer may also be a drug coating layer that contains the coating layer-forming biodegradable material and one or two or more of the aforementioned drugs at any ratio, for example, at 1:99 to 99:1 (w/w), preferably at 95 : 5 to 80 : 20 (w/w) . The method of forming the coating layer is not limited and a common coating method can be adopted as it is or after appropriately modified. Specifically, a method can be adopted in which a biodegradable material and, as needed, such a drug as mentioned above and a suitable solvent are mixed to prepare a mixture and the mixture is applied on a stent base.

Examples

The effects of the present invention will be described with reference to Examples and Comparative Examples below. In Examples, expressions "parts" or "%" are sometimes used and, unless otherwise defined, represent "parts by weight" or "% by weight". In addition, unless otherwise specified, various operations are carried out at a room temperature (25°C) .

(Example 1)

1 g of L-Lactic acid/$\varepsilon$-caprolactone copolymer of L-lactic acid : $\varepsilon$-caprolactone = 75 : 25 by mole (BMG Inc., BioDegmer (registered tradename) LCL (75 : 25), SP value: 22.6, molecular weight: 570,000), 0.1 g of pentaerythritol tetraacrylate (SP value: 21.5, PETA) (from Sigma-Aldrich) as a crosslinking agent, 0.01 g of 2-hydroxy-1-[4-(2-hydroxyethoxy)phenyl]-2-methyl-1-propano ne (2-hydroxy-1-[4-(2-hydroxyethoxy)phenyl]-2-methyl-1-propan one) (Irgacure (registered tradename) 2959, from BASF), and 29. 6 g of chloroform were mixed to prepare a polymer solution.

The polymer solution was poured into a $\phi$100-mm PFA petri dish so as not to take air bubbles and was dried with air over night at room temperature to obtain a cast film. The obtained film was irradiated with UV light of a wavelength of 365 nm from the front and back surface thereof in an integral light quantity of 3000 mJ/cm$^2$ using a UV irradiation device (VB-15201BY-A, from Ushio Inc.) to form a film (thickness: about 0.1 mm), which was then peeled from the petri dish to obtain a test film.

(Example 2)

A test film was obtained in the same manner as in Example 1 except for changing the amount of the crosslinking agent from 0.1 g to 0.2 g.

(Example 3)

A test film was obtained in the same manner as in Example 1 except for changing the amount of the crosslinking agent from 0.1 g to 0.3 g.

(Example 4)

A test film was obtained in the same manner as in Example 1 except for changing the amount of the crosslinking agent from 0.1 g to 0.4 g.

(Example 5)

A test film was obtained in the same manner as in Example 1 except for changing the amount of the crosslinking agent from 0.1 g to 0.5 g.

(Example 6)

A test film was obtained in the same manner as in Example 2 except for changing the type of the crosslinking agent from pentaerythritol tetraacrylate to dipentaerythritol hexaacrylate (SP value: 22.5, DPEHA) (from Sigma-Aldrich).

(Example 7)

A test film was obtained in the same manner as in Example 4 except for changing the type of the crosslinking agent from pentaerythritol tetraacrylate to dipentaerythritol hexaacrylate (SP value: 22.5, DPEHA) (from Sigma-Aldrich).

(Example 8)

A test film was obtained in the same manner as in Example 2 except for changing the type of the crosslinking agent from pentaerythritol tetraacrylate to ethylene glycol dimethacrylate (SP value: 18.2, EGDM) (from Sigma-Aldrich).

(Example 9)

A test film was obtained in the same manner as in Example 1 except for changing the type of the crosslinking agent from pentaerythritol tetraacrylate to ethylene glycol dimethacrylate (SP value: 18.2, EGDM) (from Sigma-Aldrich).

(Example 10)

A test film was obtained in the same manner as in Example 9 except that no initiator was added and crosslinking was performed with an electron beam of 25 kGy.

(Example 11)

A test film was obtained in the same manner as in Example 1 except for changing the L-lactic acid/ε-caprolactone copolymer of L-lactic acid : ε-caprolactone = 75 : 25% by mole to an L-lactic acid/ε-caprolactone copolymer of L-lactic acid : ε-caprolactone = 90 : 10% by mole (lactic acid-caprolactone copolymer, BMGInc., BioDegmer (registered tradename) LCL (90 : 10), (SP value: 22.9, molecular weight: 530,000).

(Example 12)

A test film was obtained in the same manner as in Example 11 except for changing the amount of the crosslinking agent from 0.1 g to 0.3 g.

(Example 13)

A test film was obtained in the same manner as in Example 11 except for changing the amount of the crosslinking agent from 0.1 g to 0.4 g.

(Example 14)

A test film was obtained in the same manner as in Example 2 except for changing the L-lactic acid/$\varepsilon$-caprolactone copolymer of L-lactic acid : $\varepsilon$-caprolactone = 75 : 25% by mole to an L-lactic acid/$\varepsilon$-caprolactone copolymer of L-lactic acid : $\varepsilon$-caprolactone = 65 : 35% by mole (lactic acid-caprolactone copolymer; BMG Inc., BioDegmer (registered tradename) LCL (65 : 35), (SP value: 22.4, molecular weight: 320,000).

(Example 15)

A test film was obtained in the same manner as in Example 14 except for changing the amount of the crosslinking agent from 0.2 g to 0.3 g.

(Example 16)

A test film was obtained in the same manner as in Example 14 except for changing the amount of the crosslinking agent from 0.2 g to 0.5 g.

(Example 17)

A test film was obtained in the same manner as in Example 4 except for changing the L-lactic acid/$\varepsilon$-caprolactone copolymer of L-lactic acid : $\varepsilon$-caprolactone = 75 : 25% by mole to an L-lactic acid/$\varepsilon$-caprolactone copolymer of L-lactic acid : $\varepsilon$-caprolactone = 60 : 40% by mole (BMG Inc., BioDegmer (registered tradename) LCL (60 : 40), SP value: 22.3, molecular weight: 370,000).

(Example 18)

A test film was obtained in the same manner as in Example 1 except for changing the type of the crosslinking agent from ethylene glycol dimethacrylate to triallyl isocyanate (SP value: 29.2, TAIC) (from Sigma-Aldrich).

(Example 19)

A test film was obtained in the same manner as in Example 18 except for changing the amount of the crosslinking agent from 0.1 g to 0.3 g.

(Example 20)

A test film was obtained in the same manner as in Example 18 except for changing the amount of the crosslinking agent from 0.1 g to 0.5 g.

(Example 21)

A test film was obtained in the same manner as in Example 10 except for changing the type of the crosslinking agent from ethylene glycol dimethacrylate to triallyl isocyanate (SP value: 29.2, TAIC) (from Sigma-Aldrich).

(Example 22)

A test film was obtained in the same manner as in Example 4 except for changing the L-lactic acid/$\varepsilon$-caprolactone copolymer of L-lactic acid : $\varepsilon$-caprolactone = 75 : 25% by mole to a DL-lactic acid/$\varepsilon$-caprolactonecopolymer of DL-lactic acid : ε-caprolactone = 90 : 10% by mole (lactic acid-caprolactone copolymer, EVONIK Industries, trade name Resomer (registered tradename) DLCL9010, Mw: 180,000, lactic acid unit : caprolactone unit = 90 : 10 (mol/mol))).

(Example 23)

A test film was obtained in the same manner as in Example 3 except for changing the L-lactic acid/ε-caprolactone copolymer of L-lactic acid : ε-caprolactone = 75 : 25% by mole to an L-lactic acid/trimethylene carbonate copolymer of L-lactic acid : trimethylene carbonate = 70 : 30% by mole (EVONIK Industries, trade name Resomer (registered tradename) LT706S, Mw: 250, 000, lactic acid unit : trimethylene carbonate unit = 70 : 30 (mol/mol))).

(Comparative Example 1)

1 g of poly-L-lactic acid (from BMG Inc., BioDegmer (registered tradename) PLLA, SP value: 23.1, weight average molecular weight: 510,000) and 29.6 g of chloroform were mixed to prepare a polymer solution. The obtained polymer solution was poured into a φ100-mm PFA petri dish so as not to take air bubbles and was dried with air at room temperature, followed by drying under reduced pressured in a vacuum oven at 120°C for 2 hours. The formed film (thickness: about 0.1 mm) was peeled from the PFA petri dish to obtain a test film.

(Comparative Example 2)

A test film was obtained in the same manner as in Comparative Example 1 except for changing poly-L-lactic acid to an L-lactic acid (75% by mole) /ε-caprolactone (25% by mole) copolymer (BMG Inc., BioDegmer (registered tradename) LCL (75 : 25), SP value: 22. 6, molecular weight: 570,000) used in Example 1 and changing the temperature and the time in the drying under reduced pressure to 80°C and 2 hours.

(Comparative Example 3)

A test film was obtained in the same manner as in Example 1 except for changing the amount of the crosslinking agent from 0.1 g to 0.6 g.

(Comparative Example 4)

A test film was obtained in the same manner as in Example 1 except for changing the amount of the crosslinking agent from 0.1 g to 0.05 g.

(Comparative Example 5)

A test film was obtained in the same manner as in Example 8 except for changing the amount of the crosslinking agent from 0.1 g to 0.05 g.

(Comparative Example 6)

A test film was obtained in the same manner as in Comparative Example 2 except for changing the L-lactic acid/ε-caprolactone copolymer (BMG Inc., BioDegmer (registered tradename) LCL (75 : 25), SP value: 22.6, molecular weight: 570,000) to the L-lactic acid/trimethylene carbonate copolymer of L-lactic acid : trimethylene carbonate = 70 : 30% by mole (EVONIK Industries, trade name Resomer (registered tradename) LT706S, Mw: 250,000, lactic acid unit : trimethylene carbonate unit = 70 : 30 (mol/mol)) used in Example 23.

[Evaluation]

<Young's modulus>

A 5B-type dumbbell test piece defined in ISO 527-2 was made with a punching die and then was subjected to a tensile test using a tensile tester equipped with a thermostatic chamber (Autograph AG-1kNIS, from Shimadzu Corporation) under an atmosphere of 37°C at a distance between chucks of 20 mm and a testing speed of 1 mm/min, and the Young's modulus (MPa) was determined from an initial slope in an elastic deformation area of the stress-strain curve.

<Recovery rate after 10 seconds, recovery rate after 20 minutes>

A 5B-type dumbbell test piece defined in ISO 527-2 was made with a punching die and then subjected to two cycles of a tensile test using a tensile tester equipped with a thermostatic chamber (Autograph AG-1kNIS, from Shimadzu Corporation) under an atmosphere of 37°C at a distance between chucks of 20 mm, a testing speed of 10 mm/min, a maximum tensile distance of 0.6 mm (length of parallel part of dumbbell test piece 12 mm × 5%), and a retention time of tensile strain of 10 seconds as shown in Fig. 2, and as shown in Fig. 3, the recovery rate was calculated as a ratio (($x_2/x_1$) × 100%) of the 2nd cycle elongation distance $x_2$ (distance from the strain-detected position to the maximum elongation position in the 2nd cycle) to the 1st cycle elongation distance $x_1$ (distance from the strain-detected position to the maximum elongation position in the 1st cycle) . Note that the stand-by time between cycles was set to 10 seconds or 20 minutes.

The recovery rate measured in this test correlates to the degree of the shape recovery of the stent. When a stent is contracted in diameter by restriction with an external force, a strain in the tensile direction is generated on an outer curving side of an apex of a bent portion. On releasing the restriction in this state, since reduction in the strain occurs as found in this test, the stent diameter is allowed to return to the diameter before contraction. In this time, the higher the recovery rate, more the strain is reduced and the closer the stent diameter becomes to the diameter before contraction. That is, the recovery rate correlates to the degree of shape recovery, and the higher the recovery rate, the higher the degree of shape recovery.

<Strain resistance property>

A 5B-type dumbbell test piece defined in ISO 527-2 was made with a punching die and then was subjected to a tensile test using a tensile tester equipped with a thermostatic chamber (Autograph AG-1kNIS, from Shimadzu Corporation) under an atmosphere of 37°C at a diameter between chucks of 20 mm and a testing speed of 10 mm/min, and whether fracture of the sample occurred was determined at 1.8-mm elongation (length of parallel part of dumbbell test piece 12 mm × 15%). A sample without fracture was rated as o and a sample with fracture was as ×.

Note that in the diameter-decreased state, in the vicinity of a bent apex, the outer curving side was elongated, that is, has a strain in the tensile direction, and the inner curving side is compressed, that is, has a strain in the compression direction. Here, the strain resistance property when designed as a stent was evaluated by whether or not fracture occurred when a strain in the tensile direction was applied. Note that a stent designed as a self-expandable stent has a tensile strain on the outer curving side and a compression strain on the inner curving side of about 10% (at most 15% or less) in a diameter-decreased state, and thus, regarding the strain resistance property, such a stent that exhibits fracture at a 15%-strain possibly undergoes fracture in a diameter decreasing operation.

<Martens hardness test>

In accordance with ISO14577-1 "Instrumented Indentation Hardness", a sheet surface was subjected to an indenter indentation test using a dynamic ultra micro hardness tester (DUH-W201S, from Shimadzu Corporation) under the following conditions: indenter: Berkovich indenter of a regular triangular pyramid shape with an intercristal angle of 115° (made of Diamond), testing force: 10 mN, loading rate: 0.473988 mN/sec, and retention time: 5 seconds, to obtain the indentation depth ($\mu$m) at this time, and the Martens hardness was determined based on the formula: [Martens hardness (N/mm$^2$)] = 1000 × [load at the indentation depth (mN)] / 26.43 × [indentation depth ($\mu$m)]$^2$.

<Gel fraction>

About 25 mg of each film was precisely weighed and was immersed in 25 ml of chloroform at 25°C for 3 hours, followed by filtration with a 200-mesh stainless wire net, and a insoluble matter on the wire net was dried in vacuum. Next, the insoluble matter was precisely weighed and the gel fraction was calculated in percentage by the following formula.

Gel fraction (%) = {weight of insoluble matter (mg) / weight of film weighed (mg)} × 100          [Math. 3]

<Biodegradable test>

A 5B-type dumbbell test piece defined in ISO 527-2 was made with a punching die, 50 mL of a phosphate buffer saline solution (pH 7.4) was placed in a 50-mL sample bottle, and the dumbbell test piece was put and completely immersed therein. The sample bottle was placed in an oven at 50°C and was allowed to stand for 2 weeks. The sample was taken out of the phosphate buffer saline solution and was immersed in ion exchange water at 37°C to wash the sample. Then, the sample was quickly subjected to a tensile test using a tensile tester equipped with a thermostatic chamber (Autograph AG-1kNIS, from Shimadzu Corporation) under an atmosphere of 37°C at a distance between chucks of 20 mm and a testing speed of 10 mm/min to measure the elongation at fracture. Separately, as a test specimen before hydrolysis, a sample was immersed in ion exchange water at 37°C for 2 hours, then was taken out, and was quickly subjected to a tensile test. Finally, a ratio of the elongation at fracture after hydrolysis to the elongation at fracture before hydrolysis ((elongation at fracture after hydrolysis/elongation at fracture before hydrolysis) $\times$ 100 (%)) was determined.

The following Table 1 shows the polymer solution compositions, the evaluation results of the above evaluation, and Tg's (°C) of the crosslinked polymers in Examples and Comparative Examples. The gel fractions in Examples were each 50% or more. For example, the gel fraction was 90% in Example 1, 52% in Example 9, and 66% in Example 11. Furthermore, any of Examples exhibits biodegradability. For example, in Example 2, the elongation at fracture before hydrolysis test was 204%, the elongation at fracture after hydrolysis test was 122%, and the ratio of the elongation at fracture after hydrolysis test to the elongation at fracture before hydrolysis test was 60%. In Comparative Example 2, the elongation at fracture before hydrolysis test was 330%, the elongation at fracture after hydrolysis test was 295%, and the ratio of the elongation at fracture after hydrolysis test to the elongation at fracture before hydrolysis test was 89%.

[Table 1-1]

| | | | Example | | | | | | | | | |
|---|---|---|---|---|---|---|---|---|---|---|---|---|
| | | | 1 | 2 | 3 | 4 | 5 | 6 | 7 | 8 | 9 | 10 |
| Copolymer | | Type | LCL7525 | LCL7525 | LCL7525 | LCL7525 | LCL7525 | LCL7525 | LCL7525 | LCL7525 | LCL7525 | LCL7525 |
| | | SP value | 22.6 | 22.6 | 22.6 | 22.6 | 22.6 | 22.6 | 22.6 | 22.6 | 22.6 | 22.6 |
| | | Molecular weight before crosslinking (Mw) | 570,000 | 570,000 | 570,000 | 570,000 | 570,000 | 570,000 | 570,000 | 570,000 | 570,000 | 570,000 |
| | | Weight | 1 g | 1 g | 1 g | 1 g | 1 g | 1 g | 1 g | 1 g | 1 g | 1 g |
| Crosslinking agent | | Type | PETA | PETA | PETA | PETA | PETA | DPEHA | DPEHA | EGDM | EGDM | EGDM |
| | | SP value | 21.5 | 21.5 | 21.5 | 21.5 | 21.5 | 22.5 | 22.5 | 18.2 | 18.2 | 18.2 |
| | | SP value difference[*1] | 1.1 | 1.1 | 1.1 | 1.1 | 1.1 | 0.1 | 0.1 | 4.4 | 4.4 | 4.4 |
| | | Weight | 0.1 g | 0.2 g | 0.3 g | 0.4 g | 0.5 g | 0.2 g | 0.4 g | 0.2 g | 0.1 g | 0.1 g |
| Proportion by weight of crosslinking agent in copolymer (%) | | | 10% | 20% | 30% | 40% | 50% | 20% | 40% | 20% | 10% | 10% |
| Initiator | | | 0.01 g | 0.01 g | 0.01 g | 0.01 g | 0.01 g | 0.01 g | 0.01 g | 0.01 g | 0.01 g | - |
| Young's modulus (MPa) | | | 563 | 992 | 1126 | 1295 | 1386 | 641 | 1417 | 735 | 706 | 592 |
| Recovery rate after 10 seconds (%) | | | 74 | 72 | 78 | 77 | 81 | 72 | 78 | 75 | 77 | 77 |
| Recovery rate after 20 minutes (%) | | | 95.5 | 90.8 | 100 | 91.7 | 90.2 | - | - | - | - | - |
| Strain resistance property | | | ○ | ○ | ○ | ○ | ○ | ○ | ○ | ○ | ○ | ○ |
| Martens hardness (N/mm$^2$) | | | 89 | 134 | 139 | 147 | 184 | 93 | 168 | 105 | 103 | 62 |
| Tg (°C) | | | - | 40 | - | - | - | - | - | - | - | - |

EP 3 653 234 B1

[Table 1-2]

| | | Example | | | | | | | | | | | | |
|---|---|---|---|---|---|---|---|---|---|---|---|---|---|---|
| | | 11 | 12 | 13 | 14 | 15 | 16 | 17 | 18 | 19 | 20 | 21 | 22 | 23 |
| Copolymer | Type | LCL9010 | LCL9010 | LCL9010 | LCL6535 | LCL6535 | LCL6535 | LCL6040 | LCL7525 | LCL7525 | LCL7525 | LCL7525 | DLCL9010 | LT706S |
| | SP value | 22.9 | 22.9 | 22.9 | 22.4 | 22.4 | 22.4 | 22.3 | 22.6 | 22.6 | 22.6 | 22.6 | 22.9 | 22.6 |
| | Molecular weight before crosslinking (Mw) | 530,000 | 530,000 | 530,000 | 320,000 | 320,000 | 320,000 | 370,000 | 570,000 | 570,000 | 570,000 | 570,000 | 180,000 | 250,000 |
| | Weight | 1 g | 1 g | 1 g | 1 g | 1 g | 1 g | 1 g | 1 g | 1 g | 1 g | 1 g | 1 g | 1 g |
| Crosslinking agent | Type | PETA | PETA | PETA | PETA | PETA | PETA | PETA | TAIC | TAIC | TAIC | TAIC | PETA | PETA |
| | SP value | 21.5 | 21.5 | 21.5 | 21.5 | 21.5 | 21.5 | 21.5 | 29.2 | 29.2 | 29.2 | 29.2 | 21.5 | 21.5 |
| | SP value difference[*1)] | 1.4 | 1.4 | 1.4 | 0.9 | 0.9 | 0.9 | 0.6 | 6.6 | 6.6 | 6.6 | 6.6 | 1.4 | 1.1 |
| | Weight | 0.1 g | 0.3 g | 0.4 g | 0.2 g | 0.3 g | 0.5 g | 0.4 g | 0.1 g | 0.3 g | 0.5 g | 0.1 g | 0.4 g | 0.3 g |
| Proportion by weight of crosslinking agent in copolymer (%) | | 10% | 30% | 40% | 20% | 30% | 50% | 40% | 10% | 30% | 50% | 10% | 40% | 30% |
| Initiator | | 0.01 g | 0.01 g | 0.01 g | 0.01 g | 0.01 g | 0.01 g | 0.01 g | 0.01 g | 0.01 g | 0.01 g | - | 0.01 g | 0.01 g |
| Young's modulus (MPa) | | 2028 | 1781 | 1640 | 511 | 655 | 880 | 502 | 615 | 689 | 506 | 501 | 938 | 1358 |
| Recovery rate after 10 seconds (%) | | 70 | 78 | 80 | 78 | 82 | 84 | 77 | 70 | 72 | 76 | 76 | 70 | 70 |
| Recovery rate after 20 minutes (%) | | - | - | - | - | - | - | - | - | - | - | - | - | - |
| Strain resistance property | | ○ | ○ | ○ | ○ | ○ | ○ | ○ | ○ | ○ | ○ | ○ | ○ | ○ |
| Martens hardness (N/mm$^2$) | | 172 | 159 | 160 | 69 | 82 | 121 | 52 | 62 | 99 | 66 | 51 | 127 | 156 |
| Tg (°C) | | - | - | - | - | - | - | - | - | - | - | - | - | - |

EP 3 653 234 B1

[Table 1-3]

| | | | Comparative Example | | | | | |
|---|---|---|---|---|---|---|---|---|
| | | | 1 | 2 | 3 | 4 | 5 | 6 |
| Copolymer | | Type | PLLA | LCL7525 | LCL7525 | LCL7525 | LCL7525 | LT706S |
| | | SP value | 23.1 | 22.6 | 22.6 | 22.6 | 22.6 | 22.6 |
| | | Molecular weight before crosslinking (Mw) | 510,000 | 570,000 | 570,000 | 570,000 | 570,000 | 250,000 |
| | | Weight | 1 g | 1 g | 1 g | 1 g | 1 g | 1 g |
| Crosslinking agent | | Type | - | - | PETA | PETA | EGDM | - |
| | | SP value | - | - | 21.5 | 21.5 | 18.2 | - |
| | | SP value difference*1) | - | - | 1.1 | 1.1 | 4.4 | - |
| | | Weight | - | - | 0.6 g | 0.05 g | 0.05 g | - |
| Proportion by weight of crosslinking agent in copolymer (%) | | | - | - | 60% | 5% | 5% | - |
| Initiator | | | - | - | 0.01 g | 0.01 g | 0.01 g | - |
| Young's modulus (MPa) | | | 2127 | 375 | 1442 | 290 | 182 | 133 |
| Recovery rate after 10 seconds (%) | | | 57 | 71 | 82 | 84 | 53 | 63 |
| Recovery rate after 20 minutes (%) | | | 75.2 | 87.8 | 86.5 | 83.8 | - | - |
| Strain resistance property | | | × | ○ | × | ○ | ○ | ○ |
| Martens hardness (N/mm$^2$) | | | 172 | 18 | 149 | 45 | 42 | 10 |
| Tg (°C) | | | 65 | 37 | - | - | - | - |

*1: Difference between solubility parameter of crosslinking agent and weighted average of solubility parameters of constitutional unit (A) and constitutional unit (B)

As can be seen from the above results, the crosslinked polymers of Examples each had a high Young's modulus, good strain resistance property, and a high 10-second recovery rate.

As can be seen from comparison of Example 10 and Example 21, when the absolute value of the difference between the solubility parameter of the crosslinking agent and the weighted average of the solubility parameters of the constitutional unit (A) and the constitutional unit (B) is 5 $(J/cm^3)^{1/2}$ or less, the Young's modulus is increased. EGDM used in Example 10 has two functional groups and has a higher Young's modulus than TAIC having three functional groups used in Example 21. That is, when TAIC or EGDM is used as the crosslinking agent, TAIC has a smaller effect of increasing the Young's modulus in spite of a larger number of functional groups (number of crosslinkable reaction points) in the added crosslinking agent, which suggests that the difference in solubility parameters is important. Similarly, in comparison of results of Example 9 and Example 18, an increased Young's modulus leads to a higher shape recovery rate. That is, when TAIC or EGDM is used as the crosslinking agent, TAIC has a smaller effect of increasing the Young's modulus and the shape recovery rate in spite of a larger number of functional groups (number of crosslinkable reaction points) in the added crosslinking agent, which suggests that the difference in solubility parameters is important. Thus, when the absolute value of the difference between the solubility parameter of the crosslinking agent and the weighted average of the solubility parameters of the constitutional unit (A) and the constitutional unit (B) is 5 $(J/cm^3)^{1/2}$ or less, the effects of the present invention (rapid shape recovery (high recovery rate) and high radial force (high Young's modulus)) are further easily obtained.

On the other hand, Comparative Example 1 in which polylactic acid is used shows a low recovery rate in spite of a high Young's modulus. In addition, Comparative Example 2 in which L-lactic acid/ε-caprolactone copolymer (75/25) that are not crosslinked was used shows a low Young's modulus. As described in [0049] of JP-T-2015-527920 (WO 2014/018123), Comparative Example 2 with an increased amount of the rubber-like polymer incorporated showed increased elasticity of the polymer and a relatively high 10-second recovery rate, while showing lowered mechanical strength.

Note that the recovery rates after 20 minutes in Examples 6 to 23 are 70% or more.

(Example 24)

A tube was formed from a material of Example 1 and was subjected to laser cutting to fabricate a self-expandable stent (thickness: 150 μm, strut width: 150 μm, outer diameter: 3.5 mm (D1), length: 18 mm) . The fabricated self-expandable stent was contracted in diameter and was inserted in a PTFE tube having an inner diameter of 1.2 mm. The tube was immersed in ion exchange water adjusted to 37°C, and the inserted self-expandable stent was released from the tube and was allowed to stand in ion exchange water at 37°C for 1 minute. Then, the stent was taken out of water and the outer diameter (D2) was measured again with a caliper to calculate the shape recovery rate ((D2/D1) $\times$ 100(%)).

As a result, the stent according to this Example showed a high shape recovery rate of 97%. This demonstrates that the stent according to the present invention can be suitably used as a self-expandable stent.

(Example 25, Comparative Example 7)

A tube was formed from a material of Example 2 or Comparative Example 2 and was subjected to laser cutting to fabricate a self-expandable stent (thickness: 100 μm, strut width: 150 μm, outer diameter: 3.2 mm, length: 11 mm).

The shape recovery rate was measured in the same manner as in Example 24. As a result, the shape recovery rates of Example 25 and Comparative Example 7 were 97%.

In addition, using the self-expandable stent, the radial force was measured in accordance with ASTM F3067-14.

(Measurement conditions)

- Measurement apparatus: Radial Force Testing System-Model RFJ from Blockwise Engineering LLC
- Measurement temperature: 37°C, speed (rate of diameter) : 0.05 mm/sec
- Measurement diameter: ϕ3.2 mm to ϕ1.5 mm
- Measurement procedure: a sample is set in the apparatus heated to 37°C. While the diameter is decreased from ϕ3.2 mm to ϕ1.5 mm at a speed of 0.05 mm/sec and then is increased from ϕ1.5 mm to ϕ3.2 mm, the radial force was measured.

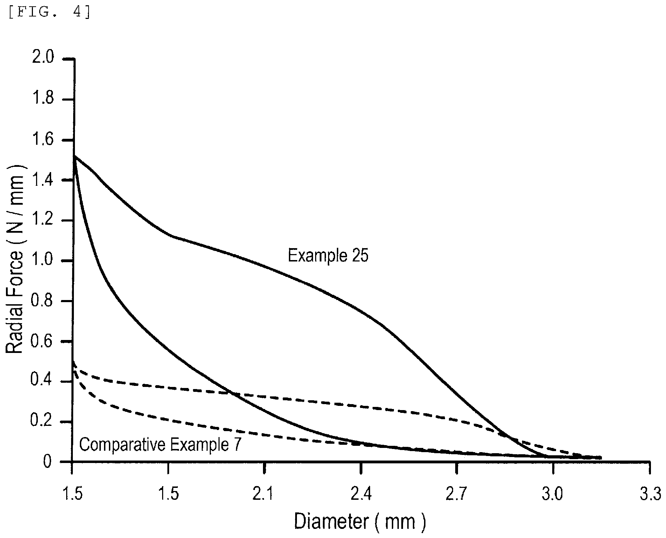

The result was shown in Fig. 4. As can be seen from Fig. 4, the stent of Example 25 showed significantly improved radial force as compared with the stent of Comparative Example 7.

The present application is based on Japanese Patent Application No. 2017-138397 filed on July 14, 2017.

**Claims**

1. A self-expandable stent comprising a crosslinked polymer comprising: a constitutional unit (A) derived from a monomer to constitute a rigid biodegradable polymer which is a homopolymer having a glass transition temperature (Tg) of 40 °C or more; a constitutional unit (B) derived from a monomer to constitute a rubber-like biodegradable polymer which is a homopolymer having a glass transition temperature (Tg) of 30 °C or lower; and a constitutional unit (C) derived from a crosslinking agent, the constitutional unit (C) being contained in an amount of 10% by weight or more and less than 60% by weight based on the total amount of the constitutional unit (A) and the constitutional unit (B).

2. The self-expandable stent according to claim 1, wherein the crosslinked polymer has a Young's modulus of 500 N/mm$^2$ or more and a recovery rate after 10 seconds of 70% or more.

3. The self-expandable stent according to claim 1 or 2, which has a Martens hardness of 50 N/mm$^2$ or more in a loading-unloading test using a nanoindenter.

4. The self-expandable stent according to any one of claims 1 to 3, wherein the crosslinked polymer is produced by polymerizing the crosslinking agent with a copolymer comprising the constitutional unit (A) derived from a monomer to constitute a rigid biodegradable polymer and the constitutional unit (B) derived from a monomer to constitute a rubber-like biodegradable polymer, and
an absolute value of a difference between a solubility parameter of the crosslinking agent and a weighted average of solubility parameters of the monomer to constitute a rigid biodegradable polymer and the monomer to constitute a rubber-like biodegradable polymer is 5 $(J/cm^3)^{1/2}$ or less, preferably 1.5 $(J/cm^3)^{1/2}$ or less.

5. The self-expandable stent according to any one of claims 1 to 4, wherein the monomer to constitute a rigid biodegradable polymer is lactic acid.

6. The self-expandable stent according to any one of claims 1 to 5, wherein the monomer to constitute a rubber-like biodegradable polymer is ε-caprolactone.

7. The self-expandable stent according to any one of claims 1 to 6, wherein the constitutional unit (B) is contained in an amount of 10 to 35% by mole based on the total amount of the constitutional unit (A) and the constitutional unit (B).

8. The self-expandable stent according to claim 7, wherein the constitutional unit (B) is contained in an amount of 20 to 30% by mole based on the total amount of the constitutional unit (A) and the constitutional unit (B).

9. The self-expandable stent according to any one of claims 1 to 8, wherein the constitutional unit (C) is contained in an amount of 30% by weight or more and less than 40% by weight based on the total amount of the constitutional unit (A) and the constitutional unit (B).

10. The self-expandable stent according to any one of claims 1 to 9, wherein the crosslinking agent is a multifunctional (meth)acrylate.

11. The self-expandable stent according to claim 10, wherein the multifunctional (meth)acrylate is a tetra- or higher functional (meth)acrylate.

12. A method of producing a self-expandable stent, comprising:

polymerizing a copolymer comprising a constitutional unit (A) derived from a monomer to constitute a rigid biodegradable polymer which is a homopolymer having a glass transition temperature (Tg) of 40 °C or more, and a constitutional unit (B) derived from a monomer to constitute a rubber-like biodegradable polymer which is a homopolymer having a glass transition temperature (Tg) of 30 °C or lower, with a crosslinking agent in an amount of 10% by weight or more and less than 60% by weight based on the amount of the copolymer to produce a crosslinked polymer; and
fabricating the stent using the crosslinked polymer.

13. The method of producing a self-expandable stent according to claim 12, wherein the copolymer has a weight average molecular weight of 100,000 to 1,000,000.

14. The method of producing a self-expandable stent according to claim 12 or 13, wherein the copolymer and the crosslinking agent are polymerized under irradiation with an ultraviolet ray.

15. The method of producing a self-expandable stent according to claim 14, wherein the copolymer comprising the constitutional unit (A) derived from a monomer to constitute a rigid biodegradable polymer and the constitutional unit (B) derived from a monomer to constitute a rubber-like biodegradable polymer and the crosslinking agent are polymerized in the presence of a photoinitiator.

**Patentansprüche**

1. Selbstexpandierbarer Stent, umfassend ein vernetztes Polymer, das umfasst: eine konstitutionelle Einheit (A), die von einem Monomer abgeleitet ist, um ein starres, biologisch abbaubares Polymer zu bilden, das ein Homopolymer ist, welches eine Glasübergangstemperatur (Tg) von 40°C oder mehr aufweist; eine konstitutionelle Einheit (B), die von einem Monomer abgeleitet ist, um ein kautschukartiges, biologisch abbaubares Polymer zu bilden, das ein Homopolymer ist, welches eine Glasübergangstemperatur (Tg) von 30 °C oder weniger aufweist; und eine konstitutionelle Einheit (C), die von einem Vernetzungsmittel abgeleitet ist, wobei die konstitutionelle Einheit (C) in einer Menge von 10 Gew.-% oder mehr und weniger als 60 Gew.-%, basierend auf der Gesamtmenge der konstitutionellen Einheit (A) und der konstitutionellen Einheit (B), enthalten ist.

**2.** Selbstexpandierbarer Stent nach Anspruch 1, wobei das vernetzte Polymer einen Elastizitätsmodul von 500 N/mm$^2$ oder mehr und eine Erholungsrate nach 10 Sekunden von 70 % oder mehr aufweist.

**3.** Selbstexpandierbarer Stent nach Anspruch 1 oder 2, der eine Martens-Härte von 50 N/mm$^2$ oder mehr in einem Belastungs-/Entlastungstest unter Verwendung eines Nanoindenters aufweist.

**4.** Selbstexpandierbarer Stent nach einem der Ansprüche 1 bis 3, wobei das vernetzte Polymer durch Polymerisation des Vernetzungsmittels mit einem Copolymer, das die konstitutionelle Einheit (A), die von einem Monomer abgeleitet ist, hergestellt wird, um ein starres, biologisch abbaubares Polymer zu bilden, und die konstitutionelle Einheit (B), die von einem Monomer abgeleitet ist, um ein kautschukartiges, biologisch abbaubares Polymer zu bilden, umfasst, und
ein absoluter Wert einer Differenz zwischen einem Löslichkeitsparameter des Vernetzungsmittels und einem gewichteten Durchschnitt der Löslichkeitsparameter des Monomers, das ein starres biologisch abbaubares Polymer bilden soll, und des Monomers, das ein kautschukartiges biologisch abbaubares Polymer bilden soll, 5 (J/cm$^3$)$^{1/2}$ oder weniger, vorzugsweise 1,5 (J/cm$^3$)$^{1/2}$ oder weniger beträgt.

**5.** Selbstexpandierbarer Stent nach einem der Ansprüche 1 bis 4, wobei das Monomer, das ein starres biologisch abbaubares Polymer bilden soll, Milchsäure ist.

**6.** Selbstexpandierbarer Stent nach einem der Ansprüche 1 bis 5, wobei das Monomer, das ein kautschukartiges, biologisch abbaubares Polymer bilden soll, ε-Caprolacton ist.

**7.** Selbstexpandierbarer Stent nach einem der Ansprüche 1 bis 6, wobei die konstitutionelle Einheit (B) in einer Menge von 10 bis 35 Mol-%, basierend auf der Gesamtmenge der konstitutionellen Einheit (A) und der konstitutionellen Einheit (B), enthalten ist.

**8.** Selbstexpandierbarer Stent nach Anspruch 7, wobei die konstitutionelle Einheit (B) in einer Menge von 20 bis 30 Mol-%, basierend auf der Gesamtmenge der konstitutionellen Einheit (A) und der konstitutionellen Einheit (B), enthalten ist.

**9.** Selbstexpandierbarer Stent nach einem der Ansprüche 1 bis 8, wobei die konstitutionelle Einheit (C) in einer Menge von 30 Gew.-% oder mehr und weniger als 40 Gew.-%, basierend auf der Gesamtmenge der konstitutionellen Einheit (A) und der konstitutionellen Einheit (B), enthalten ist.

**10.** Selbstexpandierbarer Stent nach einem der Ansprüche 1 bis 9, wobei das Vernetzungsmittel ein multifunktionelles (Meth)acrylat ist.

**11.** Selbstexpandierbarer Stent nach Anspruch 10, wobei das multifunktionelle (Meth)acrylat ein tetra- oder höherfunktionelles (Meth)acrylat ist.

**12.** Verfahren zur Herstellung eines selbstexpandierbaren Stents, umfassend:

Polymerisieren eines Copolymers, umfassend eine konstitutionelle Einheit (A), die von einem Monomer abgeleitet ist, um ein starres, biologisch abbaubares Polymer zu bilden, das ein Homopolymer ist, welches eine Glasübergangstemperatur (Tg) von 40 °C oder mehr aufweist;
und eine konstitutionelle Einheit (B), die von einem Monomer abgeleitet ist, um ein kautschukartiges, biologisch abbaubares Polymer zu bilden, das ein Homopolymer ist, welches eine Glasübergangstemperatur (Tg) von 30 °C oder weniger aufweist; mit einem Vernetzungsmittel in einer Menge von 10 Gew.-% oder mehr und weniger als 60 Gew.-%, basierend auf der Menge des Copolymers, um ein vernetztes Polymer herzustellen; und
Herstellen des Stents unter Verwendung des vernetzten Polymers.

**13.** Verfahren zur Herstellung eines selbstexpandierbaren Stents nach Anspruch 12, wobei das Copolymer ein Gewichtsmittel des Molekulargewichts von 100.000 bis 1.000.000 aufweist.

**14.** Verfahren zur Herstellung eines selbstexpandierbaren Stents nach Anspruch 12 oder 13, wobei das Copolymer und das Vernetzungsmittel unter Bestrahlung mit ultravioletter Strahlung polymerisiert werden.

**15.** Verfahren zur Herstellung eines selbstexpandierbaren Stents nach Anspruch 14, wobei das Copolymer, das die konstitutionelle Einheit (A), die von einem Monomer abgeleitet ist, um ein starres, biologisch abbaubares Polymer zu bilden, und die konstitutionelle Einheit (B), die von einem Monomer abgeleitet ist, um ein kautschukartiges, biologisch abbaubares Polymer zu bilden, und das Vernetzungsmittel umfasst, in Gegenwart eines Photoinitiators polymerisiert werden.

## Revendications

1. Endoprothèse auto-expansible comprenant un polymère réticulé comprenant : une unité constitutive (A) issue d'un monomère destiné à constituer un polymère biodégradable rigide qui est un homopolymère présentant une température de transition vitreuse (Tg) supérieure ou égale à 40 °C ; et une unité constitutive (B) issue d'un monomère destiné à constituer un polymère biodégradable du type caoutchouc qui est un homopolymère présentant une température de transition vitreuse (Tg) inférieure ou égale à 30 C ; une unité constitutive (C) issue d'un agent de réticulation, l'unité constitutive (C) étant présente en une quantité supérieure ou égale à 10 % en poids et inférieure à 60 % en poids sur la base de la quantité totale de l'unité constitutive (A) et de l'unité constitutive (B).

2. Endoprothèse auto-expansible selon la revendication 1, dans laquelle le polymère réticulé présente un module de Young supérieur ou égal à 500 N/mm$^2$ et un taux de récupération après 10 secondes supérieur ou égal à 70 %.

3. Endoprothèse auto-expansible selon la revendication 1 ou 2, qui présente une dureté Martens supérieure ou égale à 50 N/mm$^2$ lors d'un test de chargement-déchargement en utilisant un nano-indenteur.

4. Endoprothèse auto-expansible selon l'une quelconque des revendications 1 à 3, dans laquelle le polymère réticulé est produit par polymérisation de l'agent de réticulation avec un copolymère comprenant l'unité constitutive (A) issue d'un monomère destiné à constituer un polymère biodégradable rigide et l'unité constitutive (B) issue d'un monomère destiné à constituer un polymère biodégradable du type caoutchouc, et
une valeur absolue d'une différence entre un paramètre de solubilité de l'agent de réticulation et une moyenne pondérée de paramètres de solubilité du monomère destiné à constituer un polymère biodégradable rigide et du monomère destiné à constituer un polymère biodégradable du type caoutchouc est inférieure ou égale à 5 (J/cm$^3$)$^{1/2}$, de préférence inférieure ou égale à 1,5 (J/cm$^3$)$^{1/2}$.

5. Endoprothèse auto-expansible selon l'une quelconque des revendications 1 à 4, dans laquelle le monomère destiné à constituer un polymère biodégradable rigide est l'acide lactique.

6. Endoprothèse auto-expansible selon l'une quelconque des revendications 1 à 5, dans laquelle le monomère destiné à constituer un polymère biodégradable du type caoutchouc est une ε-caprolactone.

7. Endoprothèse auto-expansible selon l'une quelconque des revendications 1 à 6, dans laquelle l'unité constitutive (B) est présente en une quantité de 10 à 35 % en mole sur la base de la quantité totale de l'unité constitutive (A) et de l'unité constitutive (B).

8. Endoprothèse auto-expansible selon la revendication 7, dans laquelle l'unité constitutive (B) est présente en une quantité de 20 à 30 % en mole sur la base de la quantité totale de l'unité constitutive (A) et de l'unité constitutive (B).

9. Endoprothèse auto-expansible selon l'une quelconque des revendications 1 à 8, dans laquelle l'unité constitutive (C) est présente en une quantité supérieure ou égale à 30 % en poids et inférieure à 40 % en poids sur la base de la quantité totale de l'unité constitutive (A) et de l'unité constitutive (B).

10. Endoprothèse auto-expansible selon l'une quelconque des revendications 1 à 9, dans laquelle l'agent de réticulation est un (méth)acrylate multifonctionnel.

11. Endoprothèse auto-expansible selon la revendication 10, dans laquelle le (méth)acrylate multifonctionnel est un (méth)acrylate tétrafonctionnel ou d'une fonctionnalité supérieure.

12. Procédé de production d'une endoprothèse auto-expansible, comprenant :

la polymérisation d'un copolymère comprenant une unité constitutive (A) issue d'un monomère destiné à constituer un polymère biodégradable rigide qui est un homopolymère présentant une température de transition vitreuse (Tg) supérieure ou égale à 40 °C, et une unité constitutive (B) issue d'un monomère destiné à constituer un polymère biodégradable du type caoutchouc qui est un homopolymère présentant une température de transition vitreuse (Tg) inférieure ou égale à 30 °C, avec un agent de réticulation en une quantité supérieure ou égale à 10 % en poids et inférieure à 60 % en poids sur la base de la quantité du copolymère pour produire un polymère réticulé ; et

la fabrication de l'endoprothèse en utilisant le polymère réticulé.

**13.** Procédé de production d'une endoprothèse auto-expansible selon la revendication 12, dans lequel le copolymère présente une masse moléculaire moyenne en poids de 100 000 à 1 000 000.

**14.** Procédé de production d'une endoprothèse auto-expansible selon la revendication 12 ou 13, dans lequel le copolymère et l'agent de réticulation sont polymérisés sous rayonnement avec un rayon ultraviolet.

**15.** Procédé de production d'une endoprothèse auto-expansible selon la revendication 14, dans lequel le copolymère comprenant l'unité constitutive (A) issue d'un monomère destiné à constituer un polymère biodégradable rigide et l'unité constitutive (B) issue d'un monomère destiné à constituer un polymère biodégradable du type caoutchouc et l'agent de réticulation sont polymérisés en présence d'un photoamorceur.

[FIG. 1]

(A)

<u>10</u>

(B)

[FIG. 2]

[FIG. 3]

[FIG. 4]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015527920 T
- WO 2014018123 A
- US 2010152831 A
- US 20100262223
- US 20100262223 A
- WO 2011034009 A
- US 2012158119 A
- JP 2017138397 A

**Non-patent literature cited in the description**

- **ROBERT F FEDOR.** *Poly Eng Sci,* 1974, vol. 14 (2), 147-154